# EUROPÄISCHE PATENTSCHRIFT

(11) **EP 3 833 417 B1**
(45) Veröffentlichungstag und Bekanntmachung des Hinweises auf die Patenterteilung: **15.12.2021**
(21) Anmeldenummer: 20724781.8
(22) Anmeldetag: 04.05.2020
(51) Int. Cl.: A61M 16/04

(54) **SPRECHVENTIL**
SPEAKING VALVE
VALVE DE PHONATION

(30) Priorität: 06.05.2019 DE 102019111596
(43) Veröffentlichungstag der Anmeldung: 16.06.2021
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph
(86) Internationale Anmeldenummer: PCT/EP2020/062318
(87) Internationale Veröffentlichungsnummer: WO 2020/225212

(56) Entgegenhaltungen:
- US-A1- 2004 089 291
- US-A1- 2011 220 108
- US-A1- 2014 150 779
- US-A1- 2015 273 168
- US-B1- 6 422 235

## Beschreibung

Die Erfindung betrifft ein Sprechventil umfassend zumindest ein Gehäuse und ein Betätigungsmittel zur Betätigung des Sprechventils.

Sprechventile sind aus dem Stand der Technik bekannt. So zeigt die DE 10 2013 018 423 A1 ein Sprechventil mit einem konvexen Betätigungsmittel.

Nachteilig an den aus dem Stand der Technik bekannten Betätigungsventilen ist, dass diese unzureichend präzise bedienbar sind. Eine außermittige Krafteinleitung, meist über den Finger des Bedieners, führt bei Sprechventilen oft zu einem Kippen und in der Folge zu einem unzureichenden Verschluss des Sprechventils. Meist wird dies von dem Benutzer durch Ausübung höheren Drucks kompensiert, was aber oftmals als unangenehm empfunden wird. Geometrische Hilfestellungen, wie beispielsweise konvexe Bereiche, beeinträchtigen das Erscheinungsbild des Sprechventils negativ. Sprechventile werden zudem in der Regel als schmucklos empfunden.

Aufgabe der Erfindung ist es daher, ein Sprechventil zur Verfügung zu stellen, das die Nachteile des Standes der Technik überkommt. Insbesondere ist die Aufgabe der Erfindung ein Sprechventil zur Verfügung zu stellen, das dem Benutzer eine präzise Betätigung des Betätigungsmittels ermöglicht und bevorzugt dabei ästhetischen Gesichtspunkten entspricht.

Die Aufgabe wird erfindungsgemäß gelöst mittels eines Sprechventil umfassend zumindest ein Gehäuse und ein Betätigungsmittel zur Betätigung des Sprechventils, wobei das Betätigungsmittel eine distale Druckfläche aufweist, die eine erste Teilfläche und eine zweite Teilfläche umfasst, wobei die erste Teilfläche eine erste mittlere Rauheit aufweist und die zweite Teilfläche eine zweite mittlere Rauheit aufweist, wobei die erste mittlere Rauheit und die zweite mittlere Rauheit sich um zumindest etwa 0,1 µm unterscheiden.

Weiterhin wird die Aufgabe erfindungsgemäß gelöst mittels einer Verwendung eines Betätigungsmittels eines Sprechventils mit einer distalen Druckfläche umfassend eine erste Teilfläche und eine zweite Teilfläche, wobei die erste Teilfläche eine erste mittlere Rauheit aufweist und die zweite Teilfläche eine zweite mittlere Rauheit aufweist, wobei die erste mittlere Rauheit und die zweite mittlere Rauheit sich um zumindest etwa 0,1 µm unterscheiden, zur haptischen Führung bei Betätigung des Betätigungsmittels.

Es wird ein Sprechventil umfassend zumindest ein Gehäuse und ein Betätigungsmittel zur Betätigung des Sprechventils vorgeschlagen, wobei das Betätigungsmittel eine distale Druckfläche aufweist, die eine erste Teilfläche und eine zweite Teilfläche umfasst, wobei die erste Teilfläche eine erste mittlere Rauheit aufweist und die zweite Teilfläche eine zweite mittlere Rauheit aufweist, wobei die erste mittlere Rauheit und die zweite mittlere Rauheit sich um zumindest etwa 0,1 µm unterscheiden.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±7 %, weiter bevorzugt ±6 %, weiter bevorzugt ±5 %, weiter bevorzugt ±4 % vorgesehen.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeatmet werden kann. Beispielsweise bei laryngektomierten, also kehlkopflosen Personen, bei welchen der Kehlkopf operativ entfernt wurde, muss das Tracheostoma dauerhaft offengehalten und stabilisiert werden, wozu insbesondere Trachealkanülen, in der Regel aus einer Außen- und einer Innenkanüle bestehend, in das Tracheostoma eingesetzt werden. Aber auch der Einsatz so genannter "Tracheostoma-Button" ist möglich, insbesondere für Personen, die keine Trachealkanüle mehr benötigen.

Des Weiteren können in das Tracheostoma auch so genannte "Shunt-Ventile" eingesetzt werden, welche eine Wiederherstellung der Stimme ermöglichen. Schließlich können in das Tracheostoma auch Filtersysteme sowohl bei Tracheotomierten als auch bei Laryngektomierten eingesetzt werden. Derartige Filtersysteme bestehen insbesondere aus einem Pflaster mit einem eingesetzten Filter in einem Gehäuse oder aus einer üblicherweise selbstklebenden Basisplatte - in aller Regel aus Kunststoff gebildet, in welche Filter in einem Gehäuse unterschiedlichster Art einsetzbar sind.

Zu den Filtersystemen, welche für laryngo-tracheale Hilfsmittel wie Trachealkanülen, Shunt-Ventile, Tracheostoma-Button und Filtersysteme mit Pflaster oder Basisplatte eingesetzt werden, zählen die gattungsgemäßen Wärme- und Feuchtigkeitsaustauscher, auch "künstliche Nase" genannt. Diese dienen dazu, die bei laryngektomierten und auch tracheotomierten Menschen fehlenden Regulationsmechanismen zur Erwärmung und Befeuchtung der Atemluft nachzubilden und ein In-Kontakt-Bringen der Luftröhre mit verstärkt trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Wärme- und Feuchtigkeitsaustauscher wird die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Wärme- und Feuchtigkeitsaustauscher für Laryngektomierte und auch Tracheotomierte weisen ein Filtermaterial auf ― in aller Regel aus Papier oder Schaumstoff gebildet ― durch welches die ein- und ausgeatmete Luft geleitet wird. Beim Ausatmen hält das Filtermaterial Feuchtigkeit zurück, welche dann beim Einatmen in die Luftröhre transportiert wird. Aus dem Stand der Technik bekannte Wärme- und Feuchtigkeitsaustauscher gibt es in einer großen Vielzahl unterschiedlicher Ausbildungen für unterschiedliche Adapter. Ein Universaladapter gemäß DIN EN ISO 5356-1 weist einen Durchmesser von etwa 15 mm auf, es sind jedoch aus dem Stand der Technik auch weitere Adapter mit etwa 22 mm Durchmesser bekannt.

Ein weiteres Hilfsmittel für Laryngektomierte sind Stimmprothesen, die der Wiederherstellung der Stimme nach einer Laryngektomie dienen. Die Wiederherstellung der Stimme kann durch chirurgische Maßnahmen erfolgen. Wichtigstes Hilfsmittel dabei ist eine Stimmprothese, die auch als "Shunt-Ventil" bezeichnet wird. Mittels der Stimmprothese können Funktionen des entfernten Kehlkopfes ersetzt werden. Zum einen wird durch den Einsatz der Stimmprothese eine Luftzufuhr von der Lunge über die Speiseröhre (Ösophagus) in den Rachen ermöglicht. Die Ausatemluft wird dabei zum Sprechen verwendet. Zum anderen dichtet die Stimmprothese die Verbindung von der Speiseröhre zur Luftröhre (Trachea) beim Schlucken von Speisen und Getränken ab, wodurch der Nutzer der Stimmprothese vor einem versehentlichen Verschlucken der Nahrung geschützt ist.

Sind die Wunden nach dem operativen Eingriff geheilt, können die Menschen im Normalzustand nicht sprechen. Um ein Sprechen zu ermöglichen, ist bei Verwendung einer Stimmprothese oder der noch vorhandenen Larynx bei tracheotomierten Menschen der Luftaustritt am Hals zu verschließen, damit die Luft durch die Stimmprothese beziehungsweise die Larynx geleitet wird. Hierzu werden Sprechventile verwendet.

Das Sprechventil kann beispielsweise eine Ventilplatte als Betätigungsmittel zur Bildung einer Ventilfunktion aufweisen. In einer weiteren Ausführungsform ist vorgesehen, dass eine Deckelplatte, die beispielsweise mit einem Ventilelement zusammenwirkt, als Betätigungsmittel dient. Auch andere Ausgestaltungen zur Bildung von Betätigungsmitteln sind aus dem Stand der Technik bekannt. Da die Einzelteile von Sprechventile meist aus dünnen Kunststoff gefertigt werden, um Gewicht zu sparen, ist es von Bedeutung, dass das Betätigungsmittel exakt bedient wird, da ansonsten Einzelteile verbogen werden und das Sprechventil nicht oder nicht vollständig schließt. Auch können Querkräfte als unangenehm an einem das Tracheostoma umgebenen Narbengewebe empfunden werden. Aus dem Stand der Technik sind geometrische Gestaltungen des Betätigungsmittels zur Führung des Benutzers bekannt, die aber das Erscheinungsbild des Sprechventils negativ beeinflussen.

Vorteilhaft an dem vorgeschlagenen Sprechventil ist, dass der Benutzer anhand unterschiedlicher Rauheitswerte der zumindest zwei Teilflächen eine Rückmeldung erhält, wo er sich mit dem Finger auf dem Betätigungsmittel befindet. Weiterhin ist von Vorteil, dass durch Vorsehen unterschiedlicher Rauheitswerte der zumindest zwei Teilflächen keine optische Trennung zwischen den Teilflächen stattfindet und die Druckfläche optisch als eine einheitliche Fläche wahrgenommen werden kann.

Das Sprechventil umfasst das Betätigungsmittel, mit dem vorzugsweise mittelbar oder unmittelbar eine Ventilfunktion betätigt werden kann. Das Betätigungsmittel umfasst in einer Ausgestaltung zumindest ein Material ausgewählt aus einer Gruppe umfassend Kunststoff und/oder Metall. Kunststoffe sind für das Betätigungsmittel besonders bevorzugt. Bevorzugt ist das Betätigungsmittel gefärbt. Beispielsweise ist ein Kunststoffmaterial des Betätigungsmittels gefärbt. In einer Ausgestaltung kann mittels einer Färbung des Kunststoffmaterials ein Rauheitswert, insbesondere die erste mittlere Rauheit oder die zweite mittlere Rauheit der Druckfläche erzeugt werden. Insbesondere hat neben der Auswahl des Kunststoffes und der Ausgestaltung des Herstellungswerkzeuges die Auswahl von Additiven, die insbesondere die Farbe des Kunststoffmaterials beeinflussen, einen Einfluss auf einen Rauheitswert, insbesondere die mittlere Rauheit. In einer Ausgestaltung ist das Betätigungsmittel opak, transluzent oder transparent gefärbt. In einer weiteren Ausgestaltung ist das Betätigungsmittel einfarbig oder mehrfarbig ausgestaltet. Insbesondere kann eine Marmorierung mit zumindest zwei Farben vorgesehen sein. In einer weiteren Ausgestaltung ist vorgesehen, dass dem Kunststoffmaterial Additive zugegeben werden, die einen metallischen Effekt bringen. Solche Betätigungsmittel sind beispielsweise goldfarben, bronzefarben oder silberfarben.

Die Druckfläche ist insbesondere eine distale Fläche des Betätigungsmittels, die weiter bevorzugt von einem Rand des Gehäuses umgeben ist. In einer Ausgestaltung ist vorgesehen, dass die Druckfläche kreisrund ist. In einer weiteren Ausgestaltung ist die Druckfläche oval oder polyedrisch ausgestaltet, insbesondere rechteckig. In einer weiteren Ausgestaltung weist die Druckfläche einen konkaven und/oder konvexen Abschnitt auf. In einer weiteren Ausgestaltung ist vorgesehen, dass die erste und/oder die zweite Teilfläche zumindest teilweise konvex oder konkav ausgestaltet sind. Insbesondere ist ein mittlerer Flächenabschnitt der Druckfläche konvex oder konkav ausgestaltet.

Weiter bevorzugt ist in einer Ausgestaltung vorgesehen, dass die erste Teilfläche ein randseitiger Flächenabschnitt der Druckfläche ist. In einer Ausgestaltung ist vorgesehen, dass die erste Teilfläche und die zweite Teilfläche konzentrisch zueinander sind. Insbesondere ist die erste Teilfläche eine umlaufende randseitige, weiter bevorzugt ringförmige Fläche, beispielsweise ein Kreisring, ein ovaler Ring oder ein an die äußere Form der Druckfläche angepasster Ring. Insbesondere umgibt die erste Teilfläche die zweite Teilfläche. In einer weiteren Ausgestaltung ist vorgesehen, dass die erste Teilfläche ein mittlerer Flächenabschnitt der Druckfläche ist. Insbesondere ist die erste Teilfläche von der zweiten Teilfläche umgeben. Bevorzugt ist vorgesehen, dass die erste Teilfläche derart angeordnet ist, dass auf diese eine Betätigungskraft ausübbar ist. Insbesondere ist die erste Teilfläche kreisrund, oval oder formähnlich zur Druckfläche ausgestaltet.

In einer weiteren Ausgestaltung ist vorgesehen, dass die erste Teilfläche eine erste mittlere Rauheit Rₐ1 aufweist. Bevorzugt ist die mittlere Rauheit Rₐ1 etwa 1,4 µm bis etwa 5 µm, bevorzugt etwa 1,5 µm bis etwa 2 µm. In einer Ausgestaltung ist vorgesehen, dass die erste Teilfläche eine erste gemittelte Rautiefe R_{z}1 aufweist. Insbesondere ist die erste gemittelte Rautiefe R_{z}1 etwa 7 µm bis etwa 20 µm, bevorzugt etwa 7 µm bis etwa 15 µm, weiter bevorzugt etwa 8 µm bis etwa 11 µm.

In einer weiteren Ausgestaltung ist vorgesehen, dass die zweite Teilfläche eine Betätigungsfläche ist. Bevorzugt ist vorgesehen, dass die zweite Teilfläche derart angeordnet ist, dass auf diese eine Betätigungskraft ausübbar ist. In einer weiteren Ausgestaltung ist vorgesehen, dass die zweite Teilfläche ein mittlerer Flächenabschnitt der Druckfläche ist. Insbesondere ist die zweite Teilfläche von der ersten Teilfläche umgeben. Insbesondere ist die zweite Teilfläche kreisrund, oval oder formähnlich zur Druckfläche ausgestaltet. In einer weiteren Ausgestaltung ist vorgesehen, dass die zweite Teilfläche ein randseitiger Flächenabschnitt der Druckfläche ist. Insbesondere ist die zweite Teilfläche eine umlaufende randseitige, weiter bevorzugt ringförmige Fläche, beispielsweise ein Kreisring, ein ovaler Ring oder ein an die äußere Form der Druckfläche angepasster Ring. Insbesondere umgibt die zweite Teilfläche die erste Teilfläche.

In einer weiteren Ausgestaltung weist die zweite Teilfläche eine zweite mittlere Rauheit Rₐ2 auf. Insbesondere ist die zweite mittlere Rauheit Rₐ2 etwa 1,4 µm bis etwa 5 µm, bevorzugt etwa 1,5 µm bis etwa 2 µm. Erfindungsgemäß unterscheidet sich die erste mittlere Rauheit Rₐ1 von der zweiten mittleren Rauheit Rₐ2. Bevorzugt weist der Betrag |ΔRₐ| der Differenz von Rₐ1 und Rₐ2 zumindest etwa 0,1 µm auf. Weiter bevorzugt ist |ΔRₐ| größer etwa 0,1 µm, weiter bevorzugt ist |ΔRₐ| größer etwa 0,2 µm, weiter bevorzugt ist |ΔRₐ| größer etwa 0,3 µm, weiter bevorzugt ist |ΔRₐ| zwischen etwa 0,1 µm und etwa 5 µm, weiter bevorzugt zwischen etwa 0,1 µm und etwa 1 µm, weiter bevorzugt zwischen etwa 0,1 µm und etwa 0,5 µm, weiter bevorzugt zwischen etwa 0,2 µm und etwa 0,5 µm, weiter bevorzugt zwischen etwa 0,3 µm und 0,5 µm, weiter bevorzugt zwischen etwa 0,4 µm und etwa 0,5 µm.

Die neurologischen und biomechanischen Prozesse, auf denen eine haptische Wahrnehmung beruht, sind äußerst komplex. Feine Oberflächentexturen, wie beispielsweise eine Rauheit können nicht durch Berührung mit statischem Druck der Fingerspitzen wahrgenommen werden, sondern nur mittels dynamischer Untersuchung, wobei die Fingerspitzen über eine Oberfläche streichen und die Haut der Fingerspitzen über die Zeit unterschiedlich mechanisch verformt wird. Diese dynamischen Hautverformungen werden von den in der Epidermis und Dermis eingebetteten und auf mechanische Einwirkung ansprechenden Nervenzellen in transiente Signale umgewandelt und zum zentralen Nervensystem geleitet, um dort analysiert zu werden. Basierend auf der Analyse der transienten Signale wird die Oberflächentextur vom zentralen Nervensystem eingeordnet. Die Finger können sehr feine Strukturen wahrnehmen und schon unterschiedliche Rauigkeiten von unter etwa 0,1 µm voneinander unterscheiden. Die vorliegende Erfindung hat daher den Vorteil, dass die Druckfläche in haptisch unterschiedliche Teilbereiche unterteilt werden kann und der Benutzer optimal geführt wird, denjenigen Teilbereich zu drücken, der einen optimalen Verschluss des Sprechventils ergibt und gleichzeitig als angenehm empfunden wird.

In einer weiteren Ausgestaltung weist die zweite Teilfläche eine zweite gemittelte Rautiefe R_{z}2 auf. Bevorzugt ist die zweite gemittelte Rautiefe R_{z}2 etwa 7 µm bis etwa 20 µm, weiter bevorzugt etwa 7 µm bis etwa 15 µm, weiter bevorzugt etwa 8 µm bis etwa 11 µm.

In einer Ausgestaltung ist vorgesehen, dass die erste Teilfläche eine erste gemittelte Rautiefe R_{z}1 aufweist und die zweite Teilfläche eine zweite gemittelte Rautiefe R_{z}2 aufweist, wobei die erste gemittelte Rautiefe R_{z}1 und die zweite gemittelte Rautiefe R_{z}2 sich um zumindest etwa 0,3 µm unterscheiden. In einer Ausgestaltung unterscheidet sich die erste gemittelte Rautiefe R_{z}1 von der zweiten gemittelten Rautiefe R_{z}2. Bevorzugt unterscheiden sich sowohl die mittleren Rauheiten Ra1 und Ra2 als auch die gemittelten Rautiefen Rz1 und Rz2 voneinander. Bevorzugt weist der Betrag |ΔRz| der Differenz von Rz1 und Rz2 zumindest etwa 0,3 µm auf. In einer weiteren Ausgestaltung ist vorgesehen, dass |ΔRz| größer etwa 0,3 µm ist, weiter bevorzugt ist |ΔRz| größer etwa 0,4 µm, weiter bevorzugt ist |ΔRz| größer etwa 0,5 µm, weiter bevorzugt ist |ΔRz| zwischen etwa 0,4 µm und etwa 10 µm, weiter bevorzugt zwischen etwa 0,4 µm und etwa 5 µm, weiter bevorzugt zwischen etwa 0,4 µm und etwa 3 µm, weiter bevorzugt zwischen etwa 0,5 µm und etwa 0,3 µm

In einer Ausgestaltung ist vorgesehen, dass die erste Teilfläche und/oder die zweite Teilfläche einen Lack, beispielsweise ein Haptiklack, einen Aufkleber, beispielsweise einen bedruckten Aufkleber, oder ein sich von der jeweils anderen Teilfläche unterscheidendes Oberflächenmaterial aufweist. Wenn beispielsweise eine kleine Differenz der Rauheitswerte |ΔRₐ| und/oder |ΔRz| gewählt wird, beispielsweise |ΔRa| bis etwa 0,4 µm und/oder |ΔRz| bis etwa 2,5 µm, wird kein oder kaum ein optischer Unterscheid insbesondere in Bezug auf eine Mattheit der Oberfläche wahrgenommen. Die Betätigungsfläche wirkt einheitlich und ist nur durch haptische Unterschiede in Teilflächen aufgeteilt. In einer Ausgestaltung können sich die erste Teilfläche und die zweite Teilfläche optisch unterscheiden, beispielsweise durch insbesondere zusätzliches Aufbringen eines Logos oder eines Musters auf die erste Teilfläche oder die zweite Teilfläche.

Bevorzugt wurde eine Rauheitsmessung nach DIN EN ISO 4287 (2010-07) beziehungsweise DIN EN ISO 4288 (1998-04) / SOP 4-Tastschnitt 1 Rev. 4 durchgeführt, um die im Rahmen der Erfindung genannten Werte zu erhalten. Insbesondere wurden die folgenden Parameter für die Messung verwendet:
∘ Messgerät: MarSurf XCR20
∘ Messsoftware: MarWin 9.00-23
∘ Messtaster: BFW A 10-45-2/90°
∘ Vorschubeinheit: MarSurf GD26
∘ Messbereich: ± 250 µm
∘ Taststrecke: 5,6 mm
∘ Gesamtmessstrecke: 4,0 mm
∘ Wellenfilter: 0,8 mm
∘ Tastspitzenradius: 2 µm
∘ Tastkraft: 0,7 mN
∘ Betriebsart: Freiabtastung
∘ Anz. Einzelmessungen: 5 / Probe

Weiter bevorzugt wird je Probe ein willkürlich gewähltes Segment über einen Winkelbereich von etwa 50° bis etwa 60° mit 5 Einzelmessungen vermessen. Insbesondere erfolgt bei Proben mit einem Aufkleber die Messung vollständig auf dem Aufkleber. Weiter bevorzugt werden optisch auffällige Bereiche sowie Bereiche beispielsweise von teilweise Ablösungen des Aufklebers von den Messzonen ausgeschlossen. Bevorzugt ist ein Abstand zwischen 2 Einzelmessungen jeweils zwischen etwa 10° und etwa 15°. Bevorzugt verläuft die Tastrichtung der Einzelmessungen, bezogen auf die Probenoberfläche, von innen nach außen.

In einer ersten beispielhaften Ausgestaltungen ist vorgesehen, dass eine erste Teilfläche einer Druckfläche eine Kunststoffoberfläche mit einer metallischen Farbe ist. Weiterhin ist eine beispielhafte zweite Teilfläche mit einem Aufkleber beklebt, der beispielhaft ein aufgedrucktes Ornament aufweist. In der ersten beispielhaften Ausgestaltung weisen die Teilflächen eine Differenz der mittleren Rauheit |ΔRₐ| von etwa 0,4 µm auf. Weiterhin weisen die Teilflächen eine Differenz der gemittelten Rautiefe |ΔR_{z}| von etwa 5,6 µm auf. Mit dem oben aufgeführten Verfahren nach DIN EN ISO 4287 kann des Weiteren festgestellt werden, dass eine Differenz der gemittelten größten Profilspitze |ΔRₚ| der Teilflächen von etwa 1,9 µm vorliegt. Weiterhin ist eine Differenz der gemittelten Tiefe des größten Profiltales |ΔRᵥ| etwa 0,6 µm.

In einer zweiten beispielhaften Ausgestaltungen ist vorgesehen, dass eine erste Teilfläche einer Druckfläche eine Kunststoffoberfläche mit einer opaken türkisenen Farbe ist. Weiterhin ist eine beispielhafte zweite Teilfläche mit einem Aufkleber beklebt, der beispielhaft eine aufgedruckte Blume aufweist. In der zweiten beispielhaften Ausgestaltung weisen die Teilflächen eine Differenz der mittleren Rauheit |ΔRₐ| von etwa 0,3 µm auf. Weiterhin weisen die Teilflächen eine Differenz der gemittelten Rautiefe |ΔR_{z}| von etwa 0,3 µm auf. Mit dem oben aufgeführten Verfahren nach DIN EN ISO 4287 kann des Weiteren festgestellt werden, dass eine Differenz der gemittelten größten Profilspitze |ΔRₚ| der Teilflächen von etwa 1,4 µm vorliegt. Weiterhin ist eine Differenz der gemittelten Tiefe des größten Profiltales |ΔRᵥ| etwa 1,2 µm.

Eine beispielhafte Tabelle von Messergebnissen einer Messung nach DIN EN ISO 4287 mit den oben Aufgeführten Parametern, insbesondere gemittelt nach fünf Messdurchgängen an unterschiedlichen Stellen der Druckfläche beziehungsweise der jeweils gemessenen Teilfläche ist im Folgenden dargestellt:

| | Rₐ [µm] | ± [µm] | R_{z} [µm] | ± [µm] | Rₚ [µm] | ± [µm] | Rᵥ [µm] | ± [µm] |
|---|---|---|---|---|---|---|---|---|
| erste Teilfläche (Kunststoff metallisch) | 1,811 | 0,060 | 10,016 | 0,475 | 5,557 | 0,403 | 4,459 | 0,138 |
| erste Teilfläche (Kunststoff türkisfarben) | 1,546 | 0,060 | 9,198 | 0,446 | 4,224 | 0,144 | 4,974 | 0,508 |
| zweite Teilfläche (Ornament) | 1,635 | 0,142 | 8,737 | 0,559 | 3,614 | 0,402 | 5,123 | 0,439 |
| zweite Teilfläche (Blume) | 1,833 | 0,055 | 9,458 | 0,312 | 5,636 | 0,336 | 3,822 | 0,250 |

In einer Ausgestaltung ist vorgesehen, dass das Gehäuse ein erstes Material umfasst und das Betätigungsmittel ein zweites Material umfasst. Das Gehäuse umfasst in einer Ausgestaltung zumindest ein Gehäusematerial ausgewählt aus einer Gruppe umfassend Kunststoff oder Metall. Kunststoffe sind für das Gehäuse besonders bevorzugt. Bevorzugt ist das Gehäuse gefärbt. Beispielsweise ist ein Kunststoffmaterial des Gehäuses gefärbt. In einer Ausgestaltung ist das Gehäuse opak, transluzent oder transparent gefärbt. In einer weiteren Ausgestaltung ist das Gehäuse einfarbig oder mehrfarbig ausgestaltet. Insbesondere kann eine Marmorierung mit zumindest zwei Farben vorgesehen sein. In einer weiteren Ausgestaltung ist vorgesehen, dass dem Kunststoffmaterial des Gehäuses Additive zugegeben werden, die einen metallischen Effekt bringen. Solche Gehäuse sind beispielsweise goldfarben, bronzefarben oder silberfarben. In einer Ausgestaltung unterscheiden sich Material und/oder die Farben von Gehäuse und Betätigungsmittel. In einer weiteren Ausgestaltung sind Gehäuse und Betätigungsmittel im Wesentlichen gleich gefärbt. Durch unterschiedliche Kombinationen von Farben oder Materialien lassen sich vorteilhafter Weise Sprechventile optisch ansprechend gestalten.

Der Begriff "im Wesentlichen" gibt einen Toleranzbereich an, der für den Fachmann unter wirtschaftlichen und technischen Gesichtspunkten zu vertreten ist, sodass das entsprechende Merkmal noch als solches zu erkennen oder verwirklicht ist.

Weiterhin wird eine Verwendung eines Betätigungsmittels eines Sprechventils umfassend zumindest ein Gehäuse und ein Betätigungsmittel zur Betätigung des Sprechventils, wobei das Betätigungsmittel eine distale Druckfläche aufweist, die eine erste Teilfläche und eine zweite Teilfläche umfasst, vorgeschlagen, wobei die erste Teilfläche eine erste mittlere Rauheit aufweist und die zweite Teilfläche eine zweite mittlere Rauheit aufweist, wobei die erste mittlere Rauheit und die zweite mittlere Rauheit sich um zumindest etwa 0,1 µm unterscheiden, zur haptischen Führung bei Betätigung des Betätigungsmittels.

Weitere vorteilhafte Ausgestaltungen gehen aus der nachfolgenden Zeichnung hervor. Die dort dargestellte Weiterbildung ist jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Es zeigt

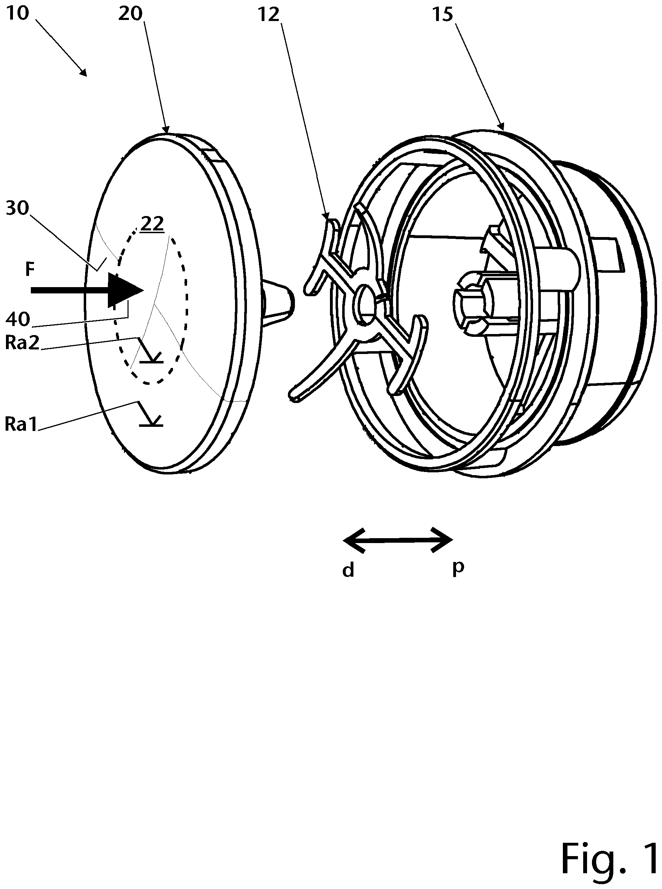

Fig. 1 ein Sprechventil in einer Explosionsansicht.

Fig. 1 zeigt ein Sprechventil 10 in einer Explosionsansicht. Zu erkennen ist ein Gehäuse 15 und ein Betätigungsmittel 20, zwischen denen ein Rückstellmittel 12 angeordnet ist. Im zusammengesetzten Zustand des Sprechventils 10 wird durch Krafteinwirkung F auf das Betätigungsmittel 20 das Betätigungsmittel 20 nach proximal p bewegt und verschließt das Gehäuse unmittelbar nach distal d. Auch andere Ausgestaltungen von Sprechventilen können vorgesehen sein, insbesondere bei denen mittels des Betätigungsmittels 20 ein Verschluss des Sprechventils 10 mittelbar erfolgt oder das Gehäuse nach proximal p verschlossen wird.

Das Betätigungsmittel 20 weist distal d eine Druckfläche 22 auf, die unterteilt ist in eine erste Teilfläche 30 und eine zweite Teilfläche 40, die in der Fig. 1 zur Verdeutlichung durch eine gestrichelte Linie getrennt sind. Die erste Teilfläche 30 weist eine erste mittlere Rauheit Rₐ1 auf. Die zweite Teilfläche 40 weist eine zweite mittlere Rauheit Rₐ2 auf. Die erste Teilfläche 30 und die zweite Teilfläche 40 unterscheiden sich insbesondere über die mittleren Rauheiten Rₐ1 und Rₐ2, die eine Differenz der mittleren Rauheit von zumindest 0,1 µm aufweisen.

Vorteilhafterweise kann mittels des Sprechventils 10 eine haptische Führung eines Benutzers für die korrekte Fingerposition auf dem Betätigungsmittel 20 erfolgen, ohne dass ein optischer Eindruck des Sprechventils gestört wird.

## Patentansprüche

1. Sprechventil (10) umfassend zumindest ein Gehäuse (15) und ein Betätigungsmittel (20) zur Betätigung des Sprechventils (10), wobei das Betätigungsmittel (20) eine distale Druckfläche (22) aufweist, die eine erste Teilfläche (30) und eine zweite Teilfläche (40) umfasst, **dadurch gekennzeichnet, dass** die erste Teilfläche (30) eine erste mittlere Rauheit (Rₐ1) aufweist und die zweite Teilfläche (40) eine zweite mittlere Rauheit (Rₐ2) aufweist, wobei die erste mittlere Rauheit (Rₐ1) und die zweite mittlere Rauheit (Rₐ2) sich um zumindest etwa 0,1 µm unterscheiden.

2. Sprechventil (10) nach Anspruch 1, **dadurch gekennzeichnet, dass** die erste Teilfläche (30) eine erste gemittelte Rautiefe (R_{z}1) aufweist und die zweite Teilfläche (40) eine zweite gemittelte Rautiefe (R_{z}2) aufweist, wobei die erste gemittelte Rautiefe (R_{z}1) und die zweite gemittelte Rautiefe (R_{z}2) sich um zumindest etwa 0,3 µm unterscheiden.

3. Sprechventil (10) nach einem oder mehreren der vorhergehenden Ansprüche, **dadurch gekennzeichnet, dass** die erste Teilfläche (30) und die zweite Teilfläche (40) konzentrisch zueinander sind.

4. Sprechventil (10) nach einem oder mehreren der vorhergehenden Ansprüche, **dadurch gekennzeichnet, dass** die erste Teilfläche (30) und/oder die zweite Teilfläche (40) einen Lack oder einen Aufkleber aufweist.

5. Sprechventil (10) nach einem oder mehreren der vorhergehenden Ansprüche, **dadurch gekennzeichnet, dass** das Gehäuse (15) ein erstes Material umfasst und das Betätigungsmittel (20) ein zweites Material umfasst.

6. Verwendung eines Betätigungsmittels (20) eines Sprechventils umfassend zumindest ein Gehäuse (15) und ein Betätigungsmittel (20) zur Betätigung des Sprechventils (10), wobei das Betätigungsmittel (20) eine distale Druckfläche (22) aufweist, die eine erste Teilfläche (30) und eine zweite Teilfläche (40) umfasst, wobei die erste Teilfläche (30) eine erste mittlere Rauheit (Rₐ1) aufweist und die zweite Teilfläche (40) eine zweite mittlere Rauheit (Rₐ2) aufweist, wobei die erste mittlere Rauheit (Rₐ1) und die zweite mittlere Rauheit (Rₐ2) sich um zumindest etwa 0,1 µm unterscheiden, zur haptischen Führung bei Betätigung des Betätigungsmittels (20).

## Claims

1. Speaking valve (10) comprising at least a housing (15) and an actuation means (20) for actuating the speaking valve (10), wherein the actuation means (20) has a distal pressure surface (22) comprising a first partial surface (30) and a second partial surface (40), **characterized in that** the first partial surface (30) has a first average roughness (Rₐ1) and the second partial surface (40) has a second average roughness (Rₐ2), wherein the first average roughness (Rₐ1) and the second average roughness (Rₐ2) differ by at least about 0.1 µm.

2. Speaking valve (10) according to claim 1, **characterized in that** the first partial surface (30) has a first average roughness depth (R_{z}1) and the second partial surface (40) has a second average roughness depth (R_{z}2), wherein the first average roughness depth (R_{z}1) and the second average roughness depth (R_{z}2) differ by at least about 0.3 µm.

3. Speaking valve (10) according to one or more of the preceding claims, **characterized in that** the first partial surface (30) and the second partial surface (40) are concentric to each other.

4. Speaking valve (10) according to one or more of the preceding claims, **characterized in that** the first partial surface (30) and/or the second partial surface (40) has a varnish or a sticker.

5. Speaking valve (10) according to one or more of the preceding claims, **characterized in that** the housing (15) comprises a first material and the actuation means (20) comprises a second material.

6. Use of an actuation means (20) of a speaking valve comprising at least a housing (15) and an actuation means (20) for actuating the speaking valve (10), wherein the actuation means (20) has a distal pressure surface (22) comprising a first partial surface (30) and a second partial surface (40), wherein the first partial surface (30) has a first average roughness (Rₐ1) and the second partial surface (40) has a second average roughness (Rₐ2), wherein the first average roughness (Rₐ1) and the second average roughness (Rₐ2) differ by at least about 0.1 µm, for haptic guidance upon actuating the actuation means (20).

## Revendications

1. Valve de phonation (10) comprenant au moins un boîtier (15) et un moyen d'actionnement (20) pour actionner ladite valve de phonation (10), ledit moyen d'actionnement (20) comportant une surface de pression distale (22) comprenant une première surface partielle (30) et une deuxième surface partielle (40) **caractérisée en ce que** ladite première surface partielle (30) présente une première rugosité moyenne (Rₐ1) et ladite seconde surface partielle (40) présente une seconde rugosité moyenne (Rₐ2), dans laquelle ladite première rugosité moyenne (Rₐ1) et ladite seconde rugosité moyenne (Rₐ2) diffèrent d'au moins environ 0,1 µm.

2. Valve de phonation (10) de la revendication 1, **caractérisée en ce que** ladite première surface partielle (30) a une première profondeur de rugosité moyenne (R_{z}1) et ladite seconde surface partielle (40) présente une seconde profondeur de rugosité moyenne (R_{z}2), dans laquelle ladite première profondeur de rugosité moyenne (R_{z}1) et ladite seconde profondeur de rugosité moyenne (R_{z}2) diffèrent d'au moins environ 0,3 µm.

3. Valve de phonation (10) selon une ou plusieurs des revendications précédentes, **caractérisée en ce que** ladite première surface partielle (30) et ladite deuxième surface partielle (40) sont concentriques les unes aux autres.

4. Valve de phonation (10) selon une ou plusieurs des revendications précédentes, **caractérisée en ce que** ladite première surface partielle (30) et/ou ladite deuxième surface partielle (40) comporte une laque ou un autocollant.

5. Valve de phonation (10) selon une ou plusieurs des revendications précédentes, **caractérisée en ce que** ledit boîtier (15) comprend un premier matériau et ledit moyen d'actionnement (20) comprend un second matériau.

6. Utilisation d'un moyen d'actionnement (20) d'une valve de phonation comprenant au moins un boîtier (15) et un moyen d'actionnement (20) pour actionner ladite valve de phonation (10), ledit moyen d'actionnement (20) comportant une surface de pression distale (22) comprenant une première surface partielle (30) et une seconde surface partielle (40), dans laquelle ladite première surface partielle (30) présente une première rugosité moyenne (Rₐ1) et ladite seconde surface partielle (40) présente une seconde rugosité moyenne (Rₐ2), dans laquelle ladite première rugosité moyenne (Rₐ1) et ladite seconde rugosité moyenne (Rₐ2) diffèrent d'au moins environ 0,1 µm, pour un guidage haptique lors dudit actionnement dudit moyen d'actionnement (20).
